(19) Europäisches Patentamt
European Patent Office
Office européen des brevets

(11) Veröffentlichungsnummer: **0 196 268**
**B1**

(12) **EUROPÄISCHE PATENTSCHRIFT**

(45) Veröffentlichungstag der Patentschrift: **27.06.90**

(51) Int. Cl.⁵: **C07J 1/00**, A61K 31/565

(21) Anmeldenummer: **86730047.7**

(22) Anmeldetag: **15.03.86**

(54) **Estriolester.**

(30) Priorität: **21.03.85 DE 3510555**

(43) Veröffentlichungstag der Anmeldung:
**01.10.86 Patentblatt 86/40**

(45) Bekanntmachung des Hinweises auf die Patenterteilung:
**27.06.90 Patentblatt 90/26**

(84) Benannte Vertragsstaaten:
**AT BE CH DE FR GB IT LI LU NL SE**

(56) Entgegenhaltungen:
**GB-A- 879 014**
**GB-A- 1 006 802**

**JOURNAL OF THE AMERICAN CHEMICAL SOCIETY, Band 82, Nr. 23, 5. Dezember 1960, Seiten 6143-6147, Washington, D.C., US; J. FISHMAN: "Rearrangements of steroidal ring D ketols"**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65(DE)**

(72) Erfinder: **Laurent, Henry, Dr., Glambecker Weg 21, D-1000 Berlin 28(DE)**
Erfinder: **Bittler, Dieter, Bölkauer Pfad 11, D-1000 Berlin 27(DE)**
Erfinder: **Beier, Sybille, Dr., Uhlandstrasse 121, D-1000 Berlin 31(DE)**
Erfinder: **Elger, Walter, Dr., Schorlemer Allee 12 B, D-1000 Berlin 33(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 196 268 B1

## Beschreibung

Die Erfindung betrifft neue Estriolester gemäß der allgemeinen Formel I

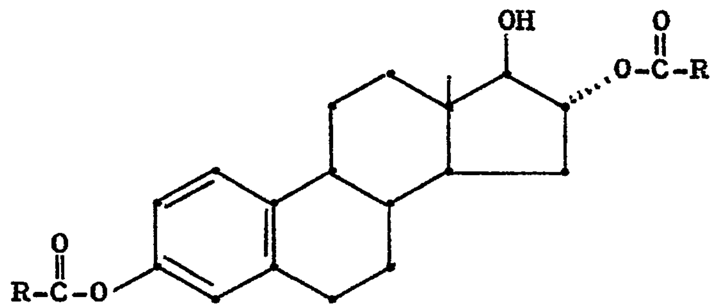

(I),

worin

R jeweils den Rest einer aliphatischen Monocarbonsäure mit 3 bis 10 Kohlenstoffatomen bedeutet, Verfahren zu ihrer Herstellung und pharmazeutische Präparate, die die neuen Estriolester enthalten.

Estriol mit der chemischen Bezeichnung 1,3,5(10)-Estratrien-3,16α,17β-triol ist ein wichtiges natürliches Östrogen.

Estriol wird auch als wirksamer Bestandteil in Präparaten zur Substitution von Östrogenen bei Östrogenmangelerscheinungen angewandt, beispielsweise bei Frauen in der Postmenopause.

Da Estriol sehr rasch aus dem Körper ausgeschieden wird, muß es in kurzen Abständen (1- bis 3mal täglich) appliziert werden.

In der Literatur sind auch einige Ester von Estriol beschrieben, beispielsweise Estriol-triester, 16,17-Diester und 16-Monoester in Chem. Pharm. Bull. 11 (1963) 510–514, ferner 3-Acetat, 3,16-Diacetat und 16,17-Diacetat in Acta Chem. Scand. 22 (1968) 254.

Aus der GB-A 1 006 802 ist das 3,16(α), 17(β)-Tripropionat des Estriols bekannt. Verglichen mit dem Estriol besitzt dieser Ester eine höhere östrogene Wirksamkeit und eine wesentlich höhere Wirkungsdauer. Die maximale Wirkung des aus dem Ester gebildeten Estriols tritt jedoch relativ frühzeitig nach der Anwendung auf, danach fällt die Wirkung wieder beträchtlich ab, etwa mit einem Drittel der Geschwindigkeit des Anstiegs.

Mit Ausnahme von Estriolsuccinat, das täglich 3mal appliziert werden muß, sind keine Estriolester medizinisch angewendet worden.

Die bisher nicht beschriebenen 3,16α-Diester des Estriols mit Monocarbonsäuren mit 3 bis 10 Kohlenstoffatomen übertreffen Estriol in der Stärke und Dauer der Östrogenwirkung.

Die Esterreste R in der allgemeinen Formel I sind vorwiegend gleich und leiten sich von einer aliphatischen Monocarbonsäure ab. Bevorzugte Esterreste R sind die von Propionsäure, Buttersäure, Isobuttersäure, Pivalinsäure, Valeriansäure, Capronsäure, Önanthsäure, Octan- und Decansäure.

Die östrogene Aktivität und die Depoteigenschaften der neuen Estriolester wurden im Vergleich zu Estriol ($E_3$) im modifizierten Uteruswachstumstest nach Rubin an ovariektomierten Ratten bestimmt (Endocrinology 49 (1951) 429 - 439).

Adulte ovariektomierte Ratten im Gewicht von ca. 150 g, 6 Tiere pro Dosisgruppe, werden einmal mit der jeweiligen Test- bzw. Referenzsubstanz (Estriol) behandelt. Der Tag der Substanzgabe gilt als Tag 1 ($d_1$) des Versuchs. Die Substanzen werden in einem Gemisch aus Benzylbenzoat + Rizinusöl im Verhältnis 4 : 6 gelöst und die Tagesdosis in einem Volumen von 0,2 ml subcutan (s.c.) appliziert. Einer Kontrollgruppe werden nur 0,2 ml Vehikel verabreicht.

Wirksame Östrogene führen bei ovariektomierten Ratten zu charakteristischen Veränderungen am Vaginalepithel. Es kommt zu einer starken Proliferation des Vaginalepithels und zur Verhornung der oberflächlichen Zellagen. Es werden täglich einmal Vaginalabstriche vorgenommen. Die Abstrichbilder werden cytologisch beurteilt.

Folgende Zyklusstadien werden unterschieden:

1 = Diöstrus (Leukozyten und kernhaltige Epithelzellen),
2 = Proöstrus (kernhaltige Epithelzellen),
3 = Östrus (kernlose Hornschollen),
4 = Metöstrus (kernlose Hornschollen, Leukozyten, Epithelzellen).

Zur Ermittlung der Dauer der Östrogenwirkung an der Vagina wird die Zeit in Tagen bestimmt, an denen der Östrus aufrechterhalten wird.

Aus Tabelle 1 ist zu entnehmen, daß nach Applikation von Estriol ($E_3$) die Tiere einen Tag im Östrus bleiben; nach Applikation equimolarer Mengen von $E_3$-Diacetat, $E_3$-Dipropionat und $E_3$-Dihexanoat hält der Östrus 4, 10 bzw. 30 Tage an.

## Tabelle 1

**Dauer der Östrogenwirkung an der Vagina nach einmaliger Injektion (s.c.) von Estriol ($E_3$) bzw. jeweils einmaliger Injektion von verschiedenen 3,16-Diestern bei ovariektomierten Ratten in equimolaren Dosen**

| Verbindung | Östrus |
|---|---|
| Estriol ($E_3$)<br>100 µg s.c. | 1 Tag |
| $E_3$-Diacetat<br>129 µg s.c. | 4 Tage |
| $E_3$-Dipropionat<br>139 µg s.c. | 10 Tage |
| $E_3$-Dihexanoat<br>168 µg s.c. | 30 Tage |

In Tabelle 2 wird der zeitliche Verlauf der Estriol ($E_3$)-Serumkonzentration in pmol/l nach einmaliger Injektion (s.c.) von Estriol ($E_3$) und jeweils einmaliger Injektion (s.c.) von verschiedenen $E_3$-3,16-Diestern bei ovariektomierten Ratten angegeben.

Am 1. Tag, 2 Stunden vor der Injektion, am 5., 10., 15., 20., 25. und 30. Tag nach der Injektion wird den Tieren Blut abgenommen, um die Serum-$E_3$-Konzentration mittels RIA (Radio Immuno Assay) zu bestimmen.

Aus Tabelle 2 geht hervor, daß nach Applikation der neuen Estriol-3,16-Diester die radioimmunologisch gemessene $E_3$-Konzentration 3 bis 7 mal höher liegt als nach equimolarer Applikation von Estriol ($E_3$) und 2 bis 4 mal höher als nach equimolarer Applikation des bekannten $E_3$-Diacetats. Bei den neuen Diestern werden erhöhte $E_3$-Konzentrationen bis zu 30 Tagen nach der Applikation beobachtet, bei Estriol und $E_3$-Diacetat nur 8 Tage lang nach der Applikation.

**T a b e l l e 2**

**Zeitlicher Verlauf der $E_3$-Serumkonzentration (pmol/l) nach einmaliger Injektion (s.c.) von Estriol ($E_3$) bzw. jeweils einmaliger Injektion (s.c.) von verschiedenen 3,16-Diestern bei ovariektomierten Ratten in equimolaren Dosen**

| Verbindung | Tag 1*) | Tag 5 | Tag 10 | Tag 15 | Tag 20 | Tag 25 | Tag 30 |
|---|---|---|---|---|---|---|---|
| Estriol ($E_3$) 100 µg s.c. | 37,2 | 63,6 | Tag 8: 57,0 | | | | |
| $E_3$-Diacetat 129 µg s.c. | 37,0 | 139,0 | 56,8 | | | | |
| $E_3$-Dipropionat 139 µg s.c. | 44,2 | 460,3 | 134,7 | 37,5 | | | |
| $E_3$-Dehexanoat 168 µg s.c. | 43,5 | 172,3 | 198,3 | 138,5 | 97,8 | 94,3 | 87,5 |
| Lösungsmittelkontrolle s.c. | 39,9 | 39,8 | 39,3 | 37,1 | 36,7 | 36,0 | 50,3 |

*) 2 Stunden vor der Injektion

Aus den tierexperimentellen Ergebnissen ist ersichtlich, daß durch Veresterung von Estriol sowohl die Wirkungsstärke als auch die Wirkungsdauer ansteigen.

Durch Veresterung von Estriol in 3,16-Stellung wird eine Stoffwechsel-stabilisierte Form eines natürlichen Östrogens erhalten, das medizinisch vielfältig anwendbar ist. Obwohl die neuen Ester auch oral verabreicht werden können, ist die parenterale Applikationsmethode bevorzugt, weil erst dann die Vorteile der neuen Ester voll in Erscheinung treten. Durch parenterale Applikation wird die erste Leberpassage und dadurch die rasche Metabolisierung vermieden. Weiterhin werden durch parenterale Applikation die schädlichen hepatischen Östrogenwirkungen vermieden, wie zum Beispiel der Anstieg der Gerinnungsfaktoren, der Anstieg der Hormontransportproteine, die Zunahme der Angiotensinogene und die Gleichgewichtsverschiebung der Lipoproteine.

Hauptanwendungsgebiete der neuen Estriolester sind die Substitution von Östrogen bei Frauen in der Postmenopause, die unter klimakterischen Ausfallserscheinungen wie Hitzewallungen, Osteoporose, Haut- und Genitalatrophie leiden, ferner die Fertilitätskontrolle bei der Frau und die gynäkologischen Indikationen, wie zum Beispiel Vaginalatrophie, Kraurosis vulvae usw.

Die zu verabreichende Menge der neuen Estriolester schwankt innerhalb eines weiten Bereichs und kann jede wirksame Menge abdecken. In Abhängigkeit des zu behandelnden Zustands, der Art und Häufigkeit der Verabreichung kann die Menge etwa 1 bis 100 mg betragen.

Die neuen Ester sind als Prodrug von Estriol zur Herstellung injizierbarer oder implantierbarer Depotpräparate besonders geeignet. Sie haben gegenüber den oral applizierbaren Präparaten auch den Vorteil, daß eine einzige Injektion für einen oder mehrere Monate ausreichend ist, während zum Beispiel Tabletten täglich eingenommen werden müssen. Die Dauer der Depotwirkung hängt von der Kettenlänge und der Menge des Esters ab sowie von der Art der Trägersubstanz, die den Wirkstoff freigibt.

Als Trägersubstanzen sind physiologisch verträgliche Verdünnungsmittel geeignet, in denen die Wirkstoffe gelöst oder suspendiert sein können. Als Verdünnungsmittel ist zum Beispiel Wasser mit oder ohne Zusatz von Elektrolytsalzen oder Verdickungsmitteln geeignet. So kann die Depotformulierung zum Beispiel eine wäßrige Mikrokristallsuspension sein.

Sehr häufig werden als Verdünnungsmittel auch Öle mit oder ohne Zusatz eines Lösungsvermittlers, eines oberflächenaktiven Stoffes, eines Suspendier- oder Emulgiermittels verwendet. Beispiele für verwendete Öle sind insbesondere Olivenöl, Erdnußöl, Baumwollsamenöl, Sojabohnenöl, Rizinusöl und Sesamöl. Beispiele für Lösungsvermittler sind insbesondere Benzylalkohol und Benzylbenzoat. Eine bevorzugte Mischung besteht aus 6 Gewichtsteilen Rizinusöl und 4 Gewichtsteilen Benzylbenzoat.

Bei der Verwendung der neuen Estriolester als Depotkontrazeptiva können die neuen Ester mit einem Depotgestagen oder einem oral zu verabreichenden Gestagen kombiniert werden. Die kombinierte Anwendung kann gleichzeitig oder zeitlich versetzt erfolgen. So kann man beispielsweise ein Depotöstrogen der allgemeinen Formel I und ein Depotgestagen zu einer 1-Monatsspritze kombinieren. Als Depotgestagen für eine ölige Lösung ist zum Beispiel Norethisteronönanthat und für eine Mikrokristallsuspension Medroxyprogesteronacetat geeignet.

Je nach der gewünschten Dauer der Wirkung können etwa 3 bis 100 mg des neuen Depotöstrogens mit 30 bis 300 mg eines Depotgestagens kombiniert werden.

Man kann auch das erfindungsgemäße Depotöstrogen injizieren und ein übliches Gestagen wie Norethisteron, Norgestrel, Levonorgestrel oder Cyproteronacetat täglich oral applizieren.

Implantationspräparate können den Wirkstoff mit inerten Materialien, zum Beispiel biologisch abbaubaren Polymeren, enthalten. Die Wirkstoffe können auch mit Silikonkautschuk zu Implantaten verarbeitet werden.

Die neuen Verbindungen der allgemeinen Formel I

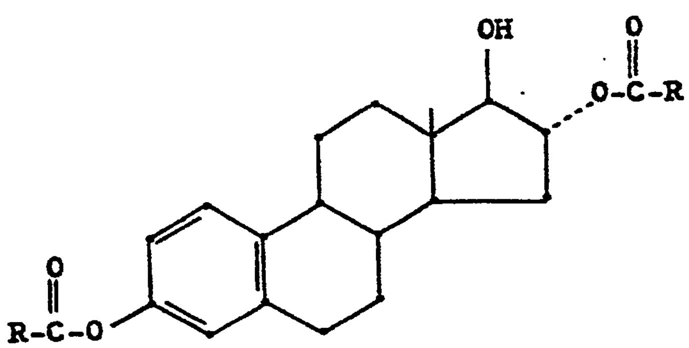

(I),

worin

R jeweils den Rest einer aliphatischen Monocarbonsäure mit 3 bis 10 Kohlenstoffatomen bedeutet, werden hergestellt, indem man 16α-Hydroxyestron der Formel II

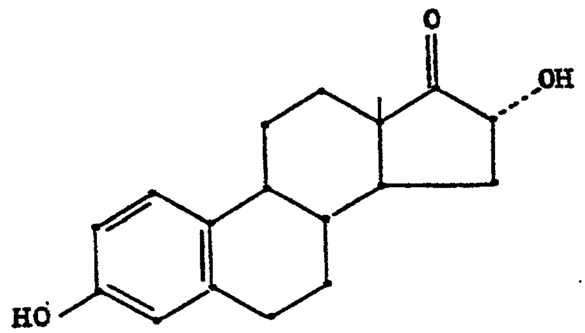

(II)

in an sich bekannter Weise in 3- und 16-Stellung verestert und anschließend das 17-Keton reduziert.

Die Veresterung der Hydroxygruppen in 3- und 16-Stellung des 16α-Hydroxyestrons erfolgt in an sich bekannter Weise mit der entsprechenden Monocarbonsäure RCOOH oder einem Derivat, insbesondere dem Anhydrid oder Chlorid der Monocarbonsäure in Gegenwart einer Base. Als Basen kommen insbesondere tertiäre Amine, wie Pyridin, 4-Dimethylaminopyridin, Collidin, Triethylamin oder Gemische aus diesen Aminen infrage.

Die sich anschließende Reduktion des 17-Ketons wird ebenfalls nach bekannten Methoden durchgeführt. Eine bevorzugte Methode ist die Reduktion mit Lithium-tri-tert.-butoxyalanat oder Natriumboranat in wasserfreiem Tetrahydrofuran bei Raumtemperatur.

Beispiel 1

a) Eine Lösung von 1,0 g 3,16α-Dihydroxy-1,3,5(10)-estratrien-17-on in 5 ml Pyridin wird mit 2,5 ml Propionsäureanhydrid versetzt und 22 Stunden bei Raumtemperatur gehalten. Das Reaktionsgemisch wird in Eiswasser gegossen, der ausgefallene Niederschlag wird abfiltriert, mit Wasser gewaschen und in Dichlormethan aufgenommen. Nach dem Trocknen über Natriumsulfat wird das Lösungsmittel im Vakuum abgedampft und der Rückstand mit einem Pentan-Diethylether-Gradienten (0 - 50 % Diethylether) an Kieselgel chromatographiert. Man erhält 640 mg 3,16α-Dipropionyloxy-1,3,5(10)-estratrien-17-on.

b) 600 mg 3,16α-Dipropionyloxy-1,3,5(10)-estratrien-17-on werden in 12 ml Tetrahydrofuran gelöst. Man versetzt die Lösung mit 600 mg Lithium-tri-tert.-butoxyalanat und rührt das Reaktionsgemisch 1 Stunde bei Raumtemperatur. Man verdünnt mit Diethylester, wäscht mit 1 M Schwefelsäure und Wasser und trocknet die organische Phase über Natriumsulfat. Anschließend wird die Lösung zur Trockne gedampft, der Rückstand wird an Kieselgel mit einem Pentan-Diethylether- Gradienten (0 - 100 % Diethylether) chromatographiert. Die das Produkt enthaltenden Fraktionen werden gemeinsam eingedampft. Der Rückstand ergibt nach dem Umkristallisieren aus Hexan 260 mg 3,16α-Dipropionyloxy-1,3,5(10)-estratrien-17β-ol vom Schmelzpunkt 118°C. $[\alpha]_D = +100°$ (in Trichlormethan).

Beispiel 2

a) Eine Lösung von 2,0 g 3,16α-Dihydroxy-1,3,5(10)-estratrien-17-on in 10 ml Pyridin und 5 ml Buttersäureanhydrid wird unter Zusatz von 100 mg 4-Dimethylaminopyridin 15 Stunden bei Raumtemperatur stehengelassen. Das Reaktionsgemisch wird mit Diethylether verdünnt, mit Wasser gewaschen, über Natriumsulfat getrocknet und eingedampft. Der Rückstand wird mit einem Pentan-Diethylether-Gradienten (0 - 30 % Diethylether) an Kieselgel chromatographiert. Es werden 1,2 g 3,16α-Dibutyryloxy-1,3,5(10)-estratrien-17-on als Öl erhalten.

b) 1,2 g 3,16α-Dibutyryloxy-1,3,5(10)-estratrien-17-on werden - wie in Beispiel 1 a) beschrieben - umgesetzt und aufgearbeitet. Man erhält nach der Chromatographie 460 mg 3,16α-Dibutyryloxy-1,3,5(10)-estratrien-17β-ol als Öl.

$[\alpha]_D = +94°$ (in Trichlormethan).

Beispiel 3

a) 2,0 g 3,16α-Dihydroxy-1,3,5(10)-estratrien-17-on werden unter den in Beispiel 2 a) beschriebenen Reaktionsbedingungen mit Valeriansäureanhydrid umgesetzt und aufgereinigt. Man erhält 1,1 g 3,16α-Divaleryloxy-1,3,5(10)-estratrien-17-on.

b) Aus 1,1 g 3,16α-Divaleryloxy-1,3,5(10)-estratrien-17-on erhält man - unter den in Beispiel 2 b) beschriebenen Bedingungen - 385 mg 3,16α-Divaleryloxy-1,3,5(10)-estratrien-17β-ol als Öl.

$[\alpha]_D = +88°$ (in Trichlormethan).

Beispiel 4

a) 1,0 g 3,16α-Dihydroxy-1,3,5(10)-estratrien-17-on werden unter den in Beispiel 2 a) beschriebenen Reaktionsbedingungen mit Capronsäureanhydrid umgesetzt und aufgereinigt. Man erhält 750 mg 3,16α-Dihexanoyloxy-1,3,5(10)-estratrien-17-on.

b) 750 mg 3,16αDihexanoyloxy-1,3,5(10)-estratrien-17-on werden unter den in Beispiel 2 b) beschriebenen Bedingungen in 240 mg 3,16α-Dihexanoyloxy-1,3,5(10)-estratrien-17β-ol überführt.
$[\alpha]_D$ = +76° (in Trichlormethan).

Beispiel 5

a) 2,0 g 3,16α-Dihydroxy-1,3,5(10)-estratrien-17-on werden - wie in Beispiel 2 a) beschrieben -, jedoch mit Decansäureanhydrid, innerhalb von 42 Stunden umgesetzt und anschließend aufgereinigt. Es werden 1,3 g 3,16α-Didecanoyloxy-1,3,5(10)-estratrien-17-on als Öl erhalten.

b) Aus 1,3 g 3,16α-Didecanoyloxy-1,3,5(10)-estratrien-17-on werden unter den in Beispiel 2 b) angegebenen Reaktionsbedingungen nach Aufreinigung 570 mg 3,16α-Didecanoyloxy-1,3,5(10)-estratrien-17β-ol als Öl erhalten.
$[\alpha]_D$ = +64,2° (in Chloroform).

**Patentansprüche für die Vertragsstaaten: BE, CH, LI, DE, FR, GB, IT, LU, NL, SE**

1.) Estriolester der allgemeinen Formel I

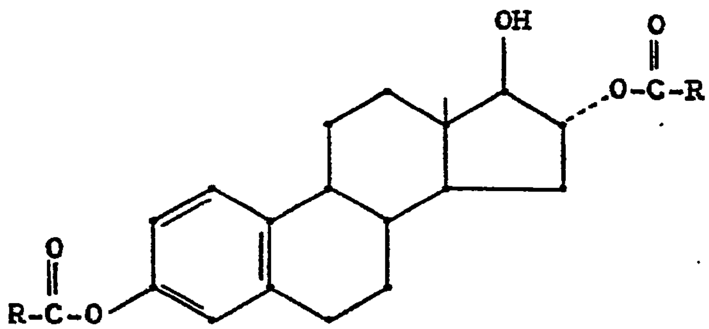

(I),

worin
R jeweils den Rest einer aliphatischen Monocarbonsäure mit 3 bis 10 Kohlenstoffatomen bedeutet.

2.) 3,16α-Dipropionyloxy-1,3,5(10)-estratrien-17β-ol.
3.) 3,16α-Dibutyryloxy-1,3,5(10)-estratrien-17β-ol.
4.) 3,16α-Divaleryloxy-1,3,5(10)-estratrien-17β-ol.
5.) 3,16α-Dihexanoyloxy-1,3,5(10)-estratrien-17β-ol.
6.) 3,16α-Didecanoyloxy-1,3,5(10)-estratrien-17β-ol.
7.) Pharmazeutische Präparate, gekennzeichnet durch den Gehalt an einer Verbindung gemäß Anspruch 1 bis 6.
8.) Depotkontrazeptiva, gekennzeichnet durch den Gehalt an einer Verbindung gemäß Anspruch 1 bis 6.
9.) Verfahren zur Herstellung neuer Estriolester der allgemeinen Formel I

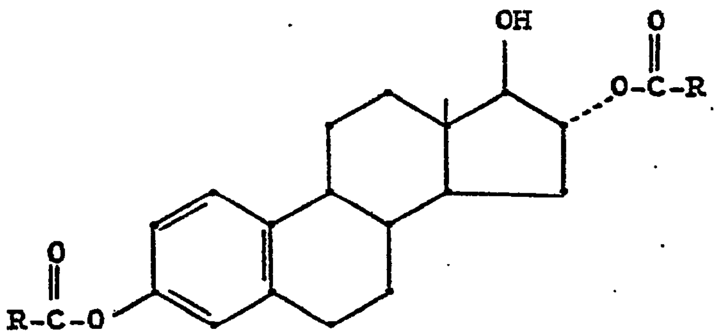

(I),

worin
R jeweils den Rest einer aliphatischen Monocarbonsäure mit 3 bis 10 Kohlenstoffatomen bedeutet,

dadurch gekennzeichnet, daß man 16α-Hydroxyestron der Formel II

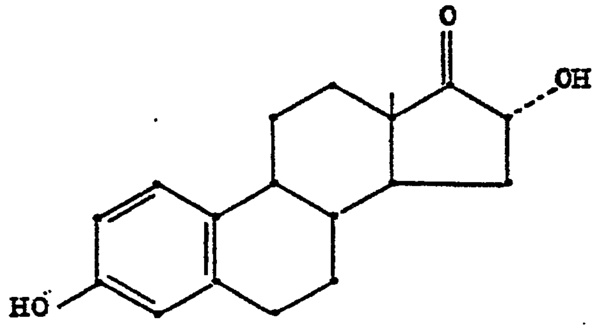

(II)

in an sich bekannter Weise in 3- und 16-Stellung verestert und anschließend das 17-Keton reduziert.

**Patentansprüche für den Vertragsstaat: AT**

Verfahren zur Herstellung neuer Estriolester der allgemeinen Formel I

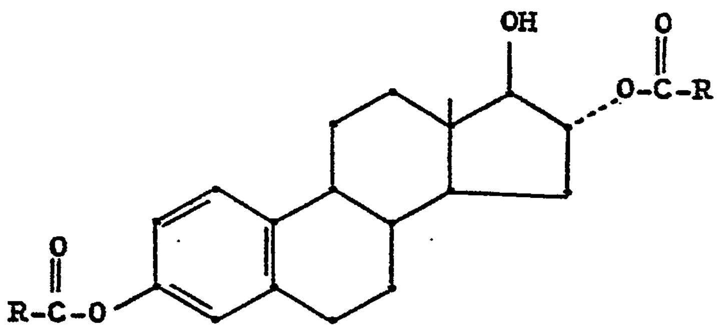

(I),

worin
R jeweils den Rest einer aliphatischen Monocarbonsäure mit 3 bis 10 Kohlenstoffatomen bedeutet,
dadurch gekennzeichnet, daß man 16α-Hydroxyestron der Formel II

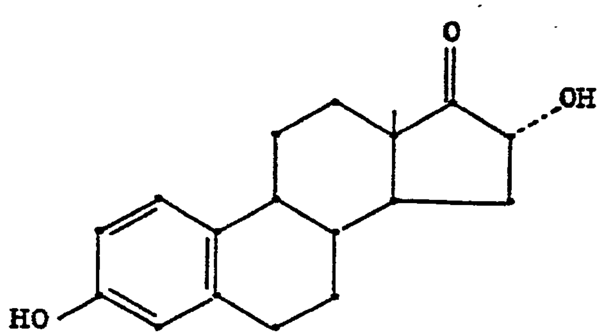

(II)

in an sich bekannter Weise in 3- und 16-Stellung verestert und anschließend das 17-Keton reduziert.

**Claims for the Contracting States: BE, CH, LI, DE, FR, GB, IT, LU, NL, SE**

1. Oestriol esters of the general formula I

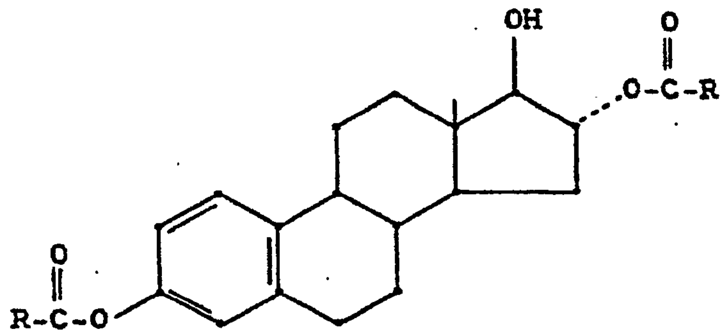

(I)

wherein
R is in each case the radical of an aliphatic monocarboxylic acid having from 3 to 10 carbon atoms.

2. 3,16α-dipropionyloxy-1,3,5(10)-oestratrien-17β-ol.
3. 3,16α-dibutyryloxy-1,3,5(10)-oestratrien-17β-ol.
4. 3,16α-divaleryloxy-1,3,5(10)-oestratrien-17β-ol.
5. 3,16α-dihexanoyloxy-1,3,5(10)-oestratrien-17β-ol.
6. 3,16α-didecanoyloxy-1,3,5(10)-oestratrien-17β-ol.
7. Pharmaceutical preparations, characterised in that they contain a compound according to claims 1 to 6.
8. Depot contraceptives, characterised in that they contain a compound according to claims 1 to 6.
9. Process for the preparation of novel oestriol esters of the general formula I

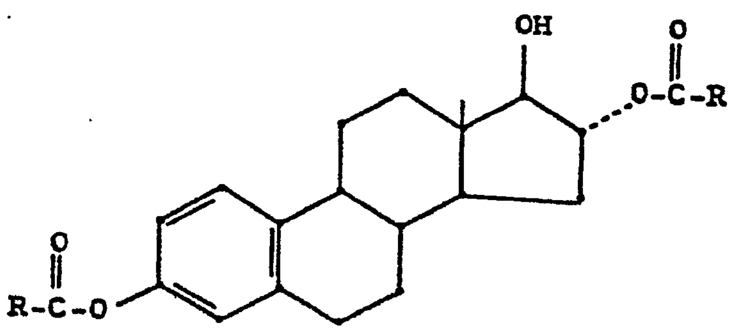

(I)

wherein
R is in each case the radical of an aliphatic monocarboxylic acid having from 3 to 10 carbon atoms, characterised in that 16α-hydroxyoestrone of the formula II

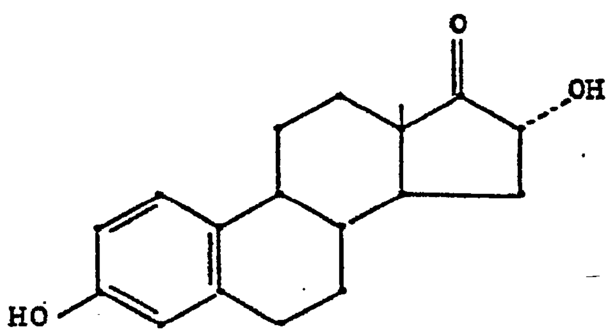

(II)

is esterified in a manner known per se in the 3- and 16-positions and then the 17-ketone is reduced.

**Claims for the Contracting State: AT**

1. Process for the preparation of novel oestriol esters of the general formula I

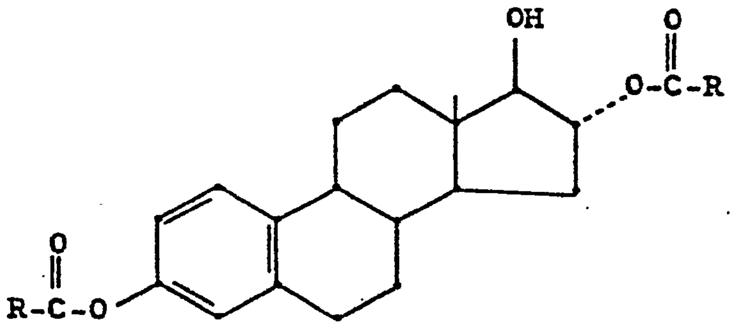

(I)

wherein
R is in each case the radical of an aliphatic monocarboxylic acid having from 3 to 10 carbon atoms, characterised in that 16α-hydroxyoestrone of the formula II

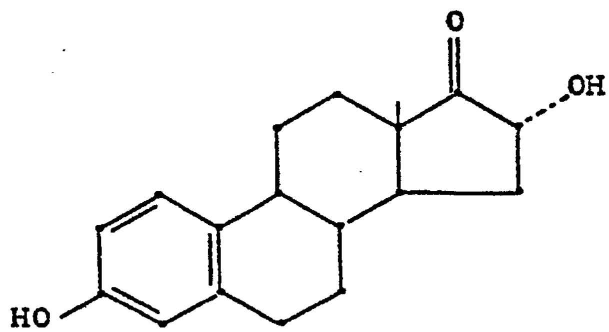

(II)

is esterified in a manner known per se in the 3- and 16-positions and then the 17-ketone is reduced.

**Revendications pour les Etats Contractants: BE, CH, LI, DE, FR, GB, IT, LU, NL, SE**

1. Esters d'oestriol répondant à la formule générale I

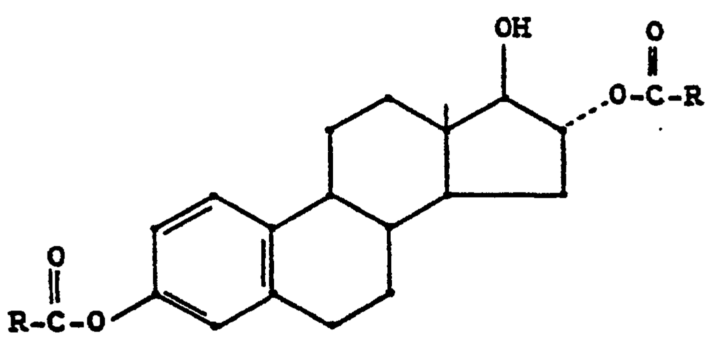

(I),

dans laquelle R représente chaque fois le radical d'un acide monocarboxylique aliphatique comportant 3 à 10 atomes de carbone.

2. 3,16α-Dipropionyloxy-1,3,5(10)-oestratrién-17β-ol.
3. 3,16α-Dibutyryloxy-1,3,5(10)-oestratrién-17β-ol.
4. 3,16α-Divaléryloxy-1,3,5(10)-oestratrién-17β-ol.
5. 3,16α-Dihexanoyloxy-1,3,5(10)-oestratrién-17β-ol.
6. 3,16α-Didécanoyloxy-1,3,5(10)-oestratrién-17β-ol.
7. Compositions pharmaceutiques, caractérisées par une teneur en un composé selon l'une des revendications 1 à 6.
8. Compositions contraceptives retard, caractérisée par une teneur en un composé selon l'une des revendications 1 à 6.

9. Procédé de préparation de nouveaux esters d'oestriol répondant à la formule générale I

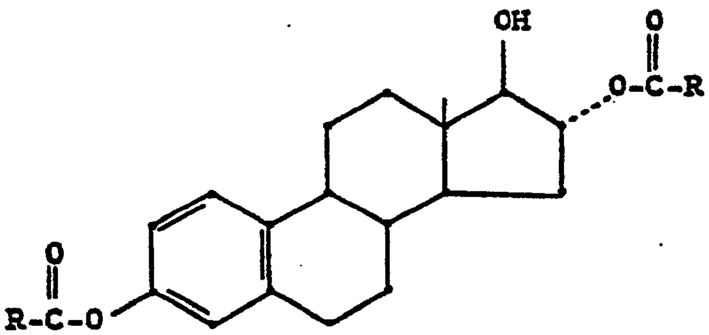

(I),

dans laquelle R représente chaque fois le radical d'un acide monocarboxylique aliphatique comportant 3 à 10 atomes de carbone, caractérisé en ce qu'on estérifie d'une manière connue en soi en position 3 et 16 une 16α-hydroxyoestrone de la formule II

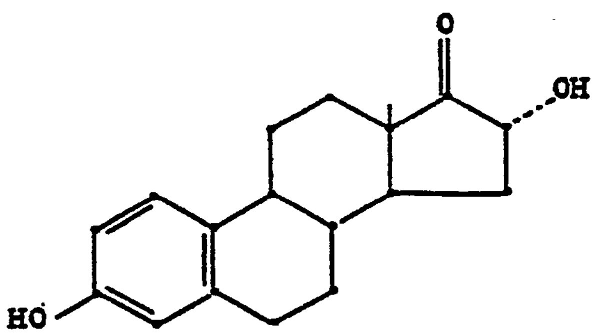

(II)

et en ce qu'ensuite on réduit la 17-cétone.

**Revendications pour l'Etat Contractant: AT**

1. Procédé de préparation de nouveaux esters d'oestriol répondant à la formule générale I

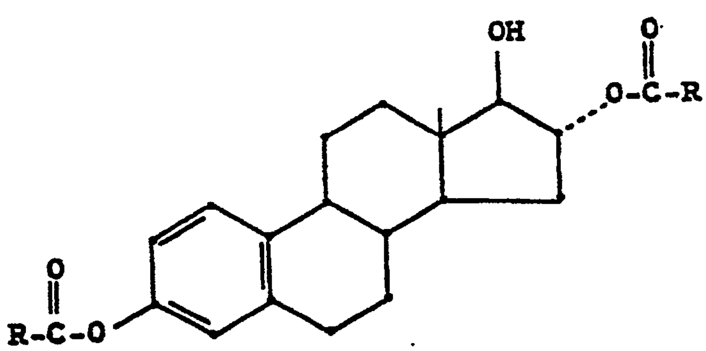

(I),

dans laquelle R représente chaque fois le radical d'un acide monocarboxylique aliphatique comportant 3 à 10 atomes de carbone, caractérisé en ce qu'on estérifie d'une manière connue en soi en position 3 et 16 une 16α-hydroxyoestrone de la formule II

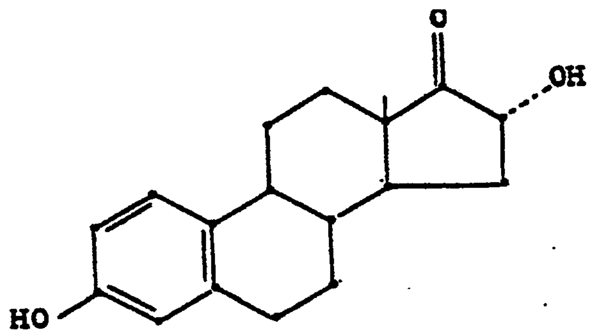

(II)

et en ce qu'ensuite on réduit la 17-cétone.